# EUROPÄISCHE PATENTSCHRIFT

(11) **EP 1 673 004 B1**
(45) Veröffentlichungstag und Bekanntmachung des Hinweises auf die Patenterteilung: **21.04.2010**
(21) Anmeldenummer: 03779973.1
(22) Anmeldetag: 18.11.2003
(51) Int. Cl.: A61B 1/018, A61B 1/005

(54) **ENDOSKOP MIT EINER FLEXIBLEN SONDE**
ENDOSCOPE COMPRISING A FLEXIBLE PROBE
ENDOSCOPE SONDE SOUPLE

(30) Priorität: 31.10.2003 DE 10351013
(43) Veröffentlichungstag der Anmeldung: 28.06.2006
(73) Patentinhaber: Polydiagnost GmbH, 85276 Pfaffenhofen (DE)
(72) Erfinder: SCHAAF, Hansgeorg, 85293 Reichertshausen (DE)
(74) Vertreter: Eisenführ, Speiser & Partner
(86) Internationale Anmeldenummer: PCT/EP2003/012897
(87) Internationale Veröffentlichungsnummer: WO 2005/051180

(56) Entgegenhaltungen:
- WO-A-93/15648
- DE-A- 3 216 178
- US-A- 4 919 112
- US-A- 5 156 590
- US-A- 5 349 942

## Beschreibung

Die Erfindung betrifft ein Endoskop mit einer flexiblen und mehrere Lumen aufweisenden Kathetersonde nach dem Oberbegriff des Patentanspruches 1.

Ein derartiges aus WO 93/15648 A bekanntes Endoskop beinhaltet eine Kathetersonde mit am proximalen Sondenende vorgesehenen Lumenausgängen und einen Handgriff mit welcher ein Optiklumen mit darin angeordnetem Optikfaserbündel verbunden ist. Das distale Ende des Optiklumens weist eine lichtdurchlässige Abdichtung auf, wobei die Optik im Optiklumen der Kathetersonde verschiebbar und aus diesem Optiklumen entfernbar angeordnet ist. Die Kathetersonde ist an ihrem proximalen Ende mit einer Kupplungseinrichtung für eine direkte Verbindung mit dem Handgriff ausgestattet. Ferner ist eine Steuereinrichtung in Form mehrerer Kabelpaare, welche mit einem am Handgriff vorgesehenen Joystick verbindbar sind vorgesehen, wodurch das distale Sondenende abgebogen werden kann.

Aus US-A-5,349,942 ist es bekannt, zum Abbiegen des distalen Kathetersondenendes einen Zugdraht zu verwenden, welcher beim Aufsetzen der Kathetersonde auf das Handstück mit einem im Handstück verschwenkbarem Betätigungshebel verbunden werden kann.

Ein aus DE 100 45 036 C1 bekanntes Endoskop besitzt eine mehrlumige Sonde sowie einen Handgriff, welcher am proximalen Sondenende vorgesehen ist. Eine Optik erstreckt sich in wenigstens einem der Sondenlumen. Ferner ist ein Arbeitslumen für ein chirurgisches Werkzeug vorgesehen. Ein Steuerelement, beispielsweise in Form eines Zugdrahtes oder eines Zugseiles ist mit dem distalen Sondenende verbunden und wird in axialer Richtung an der Sonde beweglich geführt. Auf diese Weise erreicht man ein therapeutisches Endoskop, welches beim chirurgischen Eingriff einfach bedient werden kann.

Aus US-A-4,762,120 ist ein Endoskop mit einem Handgriff und einer Katheteranordnung bekannt; bei welcher die Katheternordnung drehbar und lösbar am Handgriff befestigt wird. Im zusammengebauten Zustand ist die in der Kathetersonde vorgesehene Faseroptik mit der im Handgriff vorgesehenen Okularoptik ausgerichtet. Hierzu wird ein an der Katheteranordnung vorgesehener Optikausgang mit dem Handgriff lösbar verbunden. Die Kathetersonde besitzt weitere Lumen, deren Ausgänge am proximalen Ende außerhalb des Handgriffs liegen. Ein Steuerelement zur Führung der Sonde zum Zielort ist bei diesem Endoskop nicht vorgesehen.

Bei Endoskopen mit mehrlumigen Sonden gestaltet sich die Wartung, insbesondere die Dekontamination äußerst schwierig. Hieraus resultieren hohe Servicekosten und zwischen den jeweiligen Einsätzen entstehen hohe Ausfallzeiten, um eine sichere Dekontamination zu erreichen.

Aufgabe der Erfindung ist es daher, ein Endoskop mit steuerbarer und flexibler mehrlumiger Sonde zu schaffen, bei dem eine einfache Wartung erreicht und insbesondere bei der Dekontamination auftretende Schwierigkeiten beseitigt werden.

Diese Aufgabe wird erfindungsgemäß durch die kennzeichnenden Merkmale des Patentanspruches 1 gelöst.

In den Unteransprüchen sind vorteilhafte Weiterbildungen der Erfindung angegeben.

Bei der Erfindung wird eine starr oder drehsteif ausgebildete Führungseinrichtung für das Steuerelement am proximalen Ende der Sonde mittels eines lösbaren Verschlusses, beispielsweise Luer-Locks, drehfest mit dem Handgriff, insbesondere dem Gehäuse des Handgriffs verbunden. Die Führungseinrichtung besitzt Röhrchenform, wobei das Steuerelement durch den Röhrchenhohlraum geführt ist. Ferner wird das Steuerelement mittels eines lösbaren Befestigungsmittels, beispielsweise einer Klemmschraube, mit einem im Handgriff geführten Schieber ebenfalls lösbar verbunden. Am distalen Ende des Optiklumens in der Sonde befindet sich eine lichtdurchlässige Abdichtung bzw. Abdeckung, beispielsweise in Form einer Glas- oder Kunststoffscheibe, welche dicht in das Lumenmaterial eingesetzt ist und das Optiklumen am distalen Ende gegenüber dem Zielort hermetisch abschließt. Die lichtdurchlässige Abdeckung kann auch optische Eigenschaften, insbesondere Ausbildungseigenschaften aufweisen und beispielsweise als sen und beispielsweise als Linse ausgebildet sein. Die lichtdurchlässige Abdeckung in Form einer Scheibe oder Linse kann beispielsweise durch Kleben, Einschweißen in das insbesondere aus Kunststoff bestehende Sondenmaterial oder bei der Formgebung der Sonde durch Extrudieren, Spritzgießen oder dergleichen hermetisch dicht in die distale Öffnung des Optiklumens eingesetzt werden.

Die Optik, insbesondere Beleuchtungs- und Beobachtungsoptik, ist im Optiklumen verschiebbar und aus dem Optiklumen entfernbar angeordnet. Zum Verschieben der Optik kann am proximalen Sondenende, insbesondere am Optikausgang, durch welchen die Lichtleiter-/Faseroptik aus dem Optiklumen geführt ist, eine Schiebervorrichtung vorgesehen sein, wie sie beispielsweise aus DE 199 56 516 A1 bekannt ist. Zum Entfernen der Optik aus dem Optiklumen kann die Schiebervorrichtung lösbar am Optikausgang, beispielsweise mittels Bajonettverschluss oder Luer-Lock befestigt sein.

Anstelle eines Schiebers kann auch eine andere Einrichtung zum Längenausgleich des Optiklumens beim Verbiegen des distalen Sondenendes verwendet werden. Diese Längenausgleichseinrichtung wirkt beispielsweise mit einer bestimmten Vorspannkraft vorzugsweise einer Feder auf das Lichtleiter-/Faserbündel der Optik und drückt dieses gegen die lichtdurchlässige Abdeckung am distalen Ende des Optiklumens. Beim Abbiegen des distalen Sondenendes wird durch die federnden Vorspannkraft die Längenänderung ausgeglichen, so dass die Optik an der Abdeckung mit einer bestimmten Andrückkraft in Anlage verbleibt. Bei der Zurückbewegung des distalen Endes in die mit dem übrigen Sondenteil ausgerichtete Ausgangsposition wird der Optikstrang durch Verschieben im Optiklumen gegen die federnden Vorspannkraft in die Ausgangsposition wieder zurückgebracht.

Bei der Erfindung ist nur die Führungseinrichtung für das Steuerelement drehfest mit dem Handgriff oder dem Handgriffgehäuse verbunden. Die proximalen Ausgänge für die anderen Sondenlumen werden nicht mit dem Handgriff verbunden. Diese Sondenlumen-Ausgänge sind unabhängig vom Handgriff und außerhalb des Handgriffs an zugeordnete Endgeräte anschließbar. Beispielsweise kann die durch den Optikausgang geführte Beleuchtungsoptik, welche gegebenenfalls ferner durch die Schieber- oder Längenausgleichsvorrichtung geführt ist, an ein Beleuchtungssystem angeschlossen sein. Die Beobachtungsoptik kann an ein Okkular, welches vorzugsweise am Handgriff befestigt werden kann, angeschlossen sein. Die Beobachtungsoptik kann jedoch auch an ein Kamera-/Monitorsystem oder eine geeignete Beobachtungsseinrichtung in bekannter Weise angeschlossen sein.

Ferner kann ein Spülausgang am proximalen Ende eines Spüllumens der Sonde an ein Spül-/Absaugsystem angeschlossen sein. Außerdem kann ein Arbeitslumen oder können mehrere Arbeitslumen für ein chirurgisches Werkzeug oder für mehrere chirurgische Werkzeuge an Betätigungselemente, mit denen das jeweilige chirurgische Element betätigt wird, angeschlossen sein. Das jeweilige chirurgische Element ist hierzu entfernbar im zugeordneten Arbeitslumen geführt.

Die mehrlumige Sonde ist vorzugsweise als Einmalteil ausgebildet. Hierzu kann die Sonde durch Spritzguss- oder Extrudertechnik oder durch eine andere geeignete Formgebungstechnik hergestellt sein und aus Kunststoff bestehen. Ferner ist am proximalen Ende der Sonde ein Ansatzstück aus einem festen Material, beispielsweise Kunststoff, vorgesehen an welchem die Lumenausgänge für die mehreren Sondenlumen und der Führungsausgang für das Steuerelement vorgesehen sind. Die Lumenausgänge und der Führungsausgang können vorzugsweise Anschlusselemente für Luer-Locks, Bajonettverschlüsse oder dergleichen aufweisen oder als Kupplungsteile derartiger Verschlüsse ausgebildet sein und ebenfalls aus dem festen Material des proximalen Ansatzstückes bestehen. Vorzugsweise kann auch das proximale Ansatzstück als Spritzguss- oder Extruderteil ausgebildet sein, welches zusammen mit der flexiblen Sonde ein Einmalteil bildet.

Die Dekontamination des aus dem Arbeitslumen entfernbaren chirurgischen Werkzeugs kann in einfacher Weise durchgeführt werden. Die aus dem Optiklumen entfernte Optik ist während des chirurgischen Eingriffs nicht kontaminiert worden, da das distale Endes des Optikvolumens durch die lichtdurchlässige Abdichtung am distalen Ende gegenüber dem Zielort geschützt ist und das umgebende Sondenmaterial die Optik in ihrer Längsausdehnung schützt. Da die Sonde vorzugsweise als Einmalteil ausgebildet ist, wird für einen erneuten Einsatz eine neue, noch nicht gebrauchte Sonde mit dem Handgriff, wie oben beschrieben, verbunden.

Der Schieber, mit welchem das Steuerelement im Handgriff lösbar verbunden ist, kann vorzugsweise mittels eines Kurbeltriebs verschoben werden. Der Kurbeltrieb kann hierzu mit Hilfe eines am Handgriff vorgesehenen Betätigungselementes, beispielsweise in Form eines verschwenkbaren Hebels oder Bügels, der mit dem Kurbeltrieb fest verbunden ist, am Handgriffäußeren betätigt werden. Ferner kann eine ebenfalls am Handgriffäußeren zu betätigende Arretiereinrichtung vorgesehen sein, mit welcher der Schieber und insbesondere der Kurbeltrieb vorzugsweise stufenlos in gewünschten Positionen am Handgriff arretiert werden können.

Die Betätigungselemente für den Schieber bzw. den Kurbeltrieb und die Arretiervorrichtung sind vorzugsweise bezüglich einer durch den Handgriff verlaufenden Mittelebene symmetrisch ausgebildet. Auch das Handgriffgehäuse ist bezüglich dieser Mittelebene symmetrisch ausgebildet. Hierdurch ist gewährleistet, dass sowohl für Linkshänder als auch für Rechtshänder der Handgriff die gleiche Gestaltung aufweist. Ferner kann am Handgriff noch ein Halter für ein Okkular, welches mit der Beobachtungsoptik verbunden ist, vorgesehen sein. Dieser Halter kann durch eine Schwenklagerung, beispielsweise mit einem Kugelgelenk in jeweils gewünschte Positionen gebracht werden.

Anhand der Figuren wird an einem Ausführungsbeispiel die Erfindung noch näher erläutert.

Es zeigt
- Fig. 1: ein Ausführungsbeispiel für einen Handgriff in perspektivischer Darstellung von schräg oben her- gesehen;
- Fig. 2: eine Draufsicht auf den in Fig. 1 dargestellten Handgriff von oben;
- Fig. 3: eine Ansicht des in den Fig. 1 und 2 dargestellten Handgriffs von unten;
- Fig. 4: eine teilweise schnittbildliche Darstellung des Handgriffs der Fig. 1 bis 3 mit lösbar daran be- festigter Kathetersonde; und
- Fig. 5: aufeinanderfolgende Schritte bei der mechanischen Lithotripsie mit Hilfe eines Ausführungsbeispiels.

Das in den Figuren dargestellte Ausführungsbeispiel eines Endoskops besitzt einen Handgriff 3 und eine daran lösbar zu befestigende Kathetersonde 1. Die Sonde 1 ist als mehrlumige Sonde ausgebildet und kann beispielsweise ein Arbeitslumen oder mehrere Arbeitslumen 7 für chirurgische Werkzeuge und wenigstens ein Optiklumen 4 für eine Optik 6 aufweisen. Ferner kann ein Spüllumen 27 zum Spülen des Zielortes und zum Absaugen von Partikeln aus dem Zielort in der Sonde 1 vorgesehen sein. Für die aus Beleuchtungsoptik 22 und Beobachtungsoptik 23 bestehende Optik 6 können auch getrennte Optiklumina vorgesehen sein. Desgleichen können zum Spülen und Absaugen separate Lumina in der Sonde 1 vorgesehen sein.

Ferner beinhaltet die Kathetersonde 1 ein Steuerelement 13, beispielsweise in Form eines Zugseiles oder Zugdrahtes. Das längliche Steuerelement ist, wie beispielsweise aus DE 100 45 036 C1 bekannt ist, mit dem distalen Sondenende fest verbunden oder in der Nähe davon befestigt und erstreckt sich in axialer Richtung entlang der Sonde und ist an dieser beweglich geführt. Durch das Steuerelement kann das distale Endstück der Kathetersonde 1 gebogen werden. Das mittels eines Steuerelementes verbiegbare distale Sondenende kann auch in der Weise ausgebildet sein, wie es aus DE 201 18 886 U oder aus DE 199 28 272 A1 bekannt ist.

Die Kathetersonde 1 besteht aus einem biegbaren Material, insbesondere biokompatiblen Kunststoff. Vorzugsweise ist sie als Einmalteil ausgebildet, welches nach einem chirurgischen Einsatz vom Handgriff gelöst und entsorgt wird. Bei einem erneuten chirurgischen Eingriff wird am Handgriff 3 eine neue Kathetersonde befestigt, welche als Einmalteil steril in Bereitschaft gehalten wird.

Am proximalen Ende besitzt die Kathetersonde 1 ein Sondenansatzstück 8 aus einem starren, festen Material. Dieses Material kann ebenfalls ein Kunststoffmaterial sein. Am Sondenansatzstück 8 befinden sich proximale Lumenausgänge 9, 10 und 11 sowie eine röhrchenförmige Führungseinrichtung 12 für das Steuerelement 13. Das Steuerelement 13 wird durch den Röhrchenhohlraum geführt. Der Lumenausgang 9 ist beispielsweise dem Spüllumen 27, der Lumenausgang 10 ist beispielsweise dem Arbeitslumen 7 und der Lumenausgang 11 ist beispielsweise dem Optiklumen 4 zugeordnet. In bekannter Weise sind die Ausgänge mit Kupplungselementen ausgestattet, beispielsweise für einen Bajonettverschluss, Luer-Lock oder mit ähnlichen Kupplungs- und Anschlussstücken.

Die Kathetersonde 1 wird über die starre, röhrchenförmige Führungseinrichtung 12 für das Steuerelement 13 drehfest mit dem Handgriff 3, insbesondere dem Handgriffgehäuse verbunden. Hierzu besitzt der Handgriff an seinem vorderen Ende ein Verschlussstück, welches mit einem an der Führungseinrichtung 12 vorgesehenen Verschlussstück einen lösbaren Verschluss 2 beim Zusammenbau bildet. Der lösbare Verschluss 2 kann als Bajonettverschluss oder Luer-Lock ausgebildet sein. Durch den lösbaren Verschluss 2 wird eine drehfeste Verbindung zwischen dem Handgriff 3 und der Kathetersonde 1 gebildet. Auf diese Weise werden Drehbewegungen des Handgriffes 3 auf die Kathetersonde 1 und insbesondere auf den distalen Sondenbereich 28 mit gegebenenfalls abgebogenem distalen Sondenende übertragen. Vorzugsweise kann hierzu, wie aus DE 100 45 036 C1 bekannt, das Steuerelement 13 in seiner Längsachse drehsteif ausgebildet sein, so dass Drehungen des Handgriffes 3 drehwinkelgerecht bis zum distalen Ende der Kathetersonde 1 übertragen werden. Geeignete steuerbare distale Sondenenden sind ferner in DE 201 18 886 oder DE 199 28 272 A1 beschrieben.

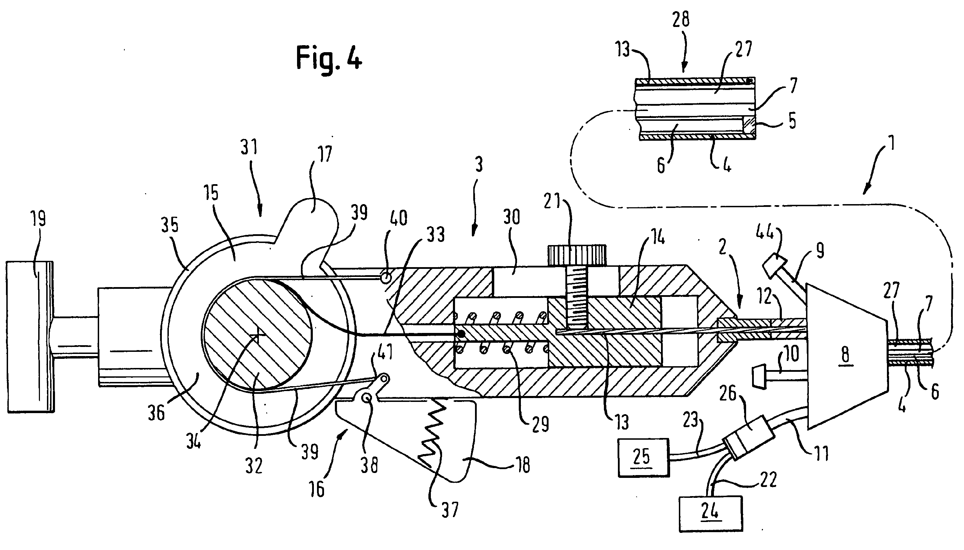

Wie insbesondere aus Fig. 4 zu ersehen ist, wird das proximale Ende des durch die röhrchenförmige Führungseinrichtung 12 geführten Steuerelementes 13 mittels eines lösbaren Befestigungsmittels 21, beispielsweise in Form einer Klemmschraube, fest mit einem in axialer Richtung verschiebbar gelagerten Schieber 14 verbunden. Der Schieber 14 ist drehfest um die Gehäuseachse des Handgriffes 3 im Handgriffgehäuse gelagert. Auf diese Weise erreicht man eine drehfeste Verbindung zwischen dem Handgriff 3 bzw. dem Gehäuse des Handgriffes 3 und dem Steuerelement 13. Aufgrund der Formgebung, beispielsweise des Sondenansatzstückes 8 oder aufgrund einer Markierung an der Kathetersonde, beispielsweise am Sondenansatzstück 8 kann die Winkelposition der Kathetersonde, insbesondere die Position des distalen Sondenendes 28 und die Drehwinkelposition der Optik 6 um die Sondenachse erkannt werden.

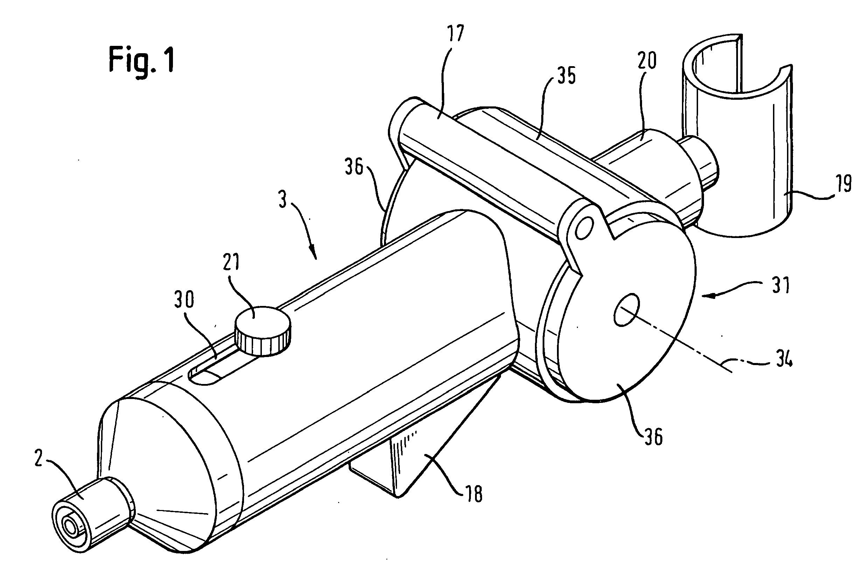

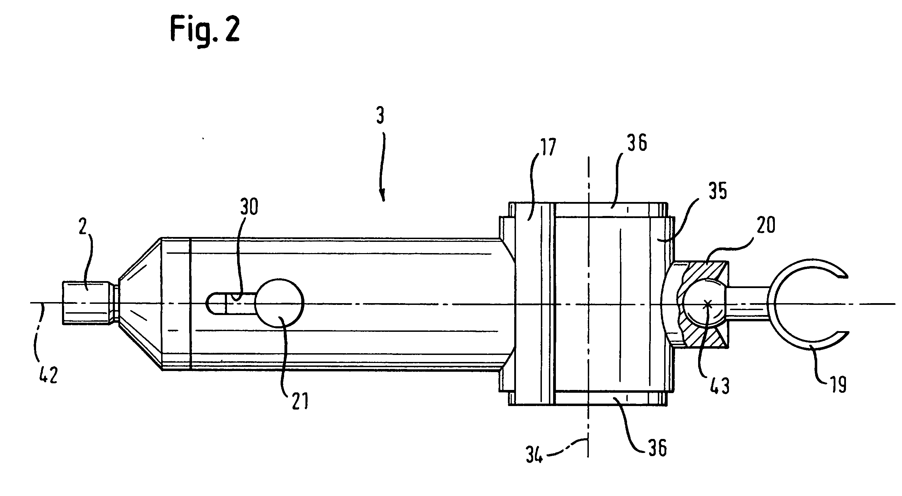

Der Schieber 14 wird im Gehäuse des Handgriffs 3 axial beweglich gegen die Vorspannkraft, beispielsweise einer Vorspannfeder 29 in axialer Richtung beweglich geführt. Die Vorspannkraft wirkt dabei in Richtung zum vorderen (distalen) Ende des Handgriffs 3 hin. In der vordersten Position des Schiebers wird das distale Sondenende 28 gegenüber dem übrigen Sondenbereich nicht abgebogen. Beim Verschieben des Schiebers 14 gegen die Vorspannkraft der Feder 29 in Richtung zum rückwärtigen (proximalen) Ende des Handgriffs 3 hin, wird diese Bewegung über das Steuerelement 13 auf das distale Sondenende 28 übertragen und das distale Sondenende in Abhängigkeit von der Strecke der Verschiebebewegung abgebogen. Die Betätigung des Schiebers kann beispielsweise mittels eines mit dem Befestigungsmittel, beispielsweise der Klemmschraube 21 verbundenen Betätigungselements erfolgen. Beispielsweise kann der Kopf der Klemmschraube so bemessen sein, dass er als Betätigungselement wirkt. Es kann jedoch auch ein zusätzliches Betätigungselement vorgesehen sein. Das Betätigungselement kann hierzu durch einen in axialer Richtung des Handgriffs 3 verlaufenden Längsschlitz 30 im Handgriffgehäuse ragen, wie das für die als lösbares Befestigungsmittel wirkende Klemmschraube 21 in den Fig. 1 und 2 dargestellt ist. Der Längsschlitz 30 kann an seinen beiden Enden Anschläge zur Begrenzung der Bewegung des Schiebers 14 und der axialen Steuerbewegung des Steuerelementes 13 bilden. Bei dem dargestellten lösbaren Befestigungsmittel 21 in Form der Klemmschraube, welche durch den Längsschlitz 30 des Handgriffgehäuses ragt, wird eine erleichterte Befestigung des proximalen Endes des Steuerelementes 13 am Schieber 14 erreicht.

Beim dargestellten Ausführungsbeispiel ist zur Erzeugung der axialen Schieberbewegung ein Kurbeltrieb 31 vorgesehen, mit welchem eine am Handgriffäußeren erzeugte Schwenkbewegung bzw. Drehbewegung in die axiale, linear verlaufende Schieberbewegung umgesetzt wird. Der beim Ausführungsbeispiel zum Einsatz kommende Kurbeltrieb 31 besitzt einen Wickelkörper 32, auf welchen ein Zugmittel 33 in Form eines Seiles, eines Drahtes oder eines Bandes aufwickelbar ist. Das eine Ende des Zugmittels 33 ist mit dem Wickelkörper 32 und das andere Ende des Zugmittels 32 ist mit dem Schieber 14 fest verbunden. Mit dem Wickelkörper 32 ist ein Betätigungselement 17, welches außen am Handgriff 3 schwenkbar gelagert ist, fest verbunden. Das Betätigungselement 17 besitzt beim dargestellten Ausführungsbeispiel einen parallel zur Achse 32 des Wickelkörpers sich erstreckenden Betätigungsbügel. Die Achse 34 und das Betätigungselement 17 liegen dabei quer (senkrecht) zur Längsausdehnung des länglich, insbesondere rohrförmig ausgebildeten Handgriffgehäuses. Das Betätigungselement 17 ist mit beiden Enden des rollenförmigen Wickelkörpers 32 fest verbunden. Hierzu kann der Wickelkörper 32 an seinen beiden Enden über seine hohlzylindrische Lagerung 35 am Gehäuse hinausragen. Es ist jedoch auch möglich, über fest mit den beiden Enden des Wickelkörpers 32 verbundene Endscheiben 36 das bügelförmige Betätigungselement 17 mit dem Wickelkörper 32 zu verbinden. In Abhängigkeit vom Schwenkwinkelbereich des Betätigungselementes 17 und damit des Wickelkörpers 32 um seine Achse 34 wird der Schieber 14 im Gehäuse axial verschoben. Wie schon erläutert, wird diese Schieberbewegung auf das distale Sondenende 28 zum Abbiegen des Sondenendes 28 gegenüber dem übrigen Sondenteil übertragen.

Die Schieberbewegung und damit das Abbiegen des distalen Sondenendes 28 kann in verschiedenen Positionen mit Hilfe einer Arretiereinrichtung 16 arretiert werden. Beim dargestellten Ausführungsbeispiel wirkt die Arretiereinrichtung 16 auf den Kurbeltrieb 31, insbesondere die Stellung des Wickelkörpers 32. Es ist jedoch auch möglich, eine Arretiereinrichtung vorzusehen, welche unmittelbar auf den Schieber 14, beispielsweise in Form einer Klemmschraube, Klemmhebels oder dergleichen wirkt.

Die im Ausführungsbeispiel in Fig. 4 dargestellte Arretiereinrichtung 16 beinhaltet ein Reibband 39, welches teilweise, beispielsweise mit einem Umschlingungswinkel von ca. 180° um den Wickelkörper 32 geschlungen ist. Das eine Bandende 40 ist am Handgriffgehäuse fixiert und das andere Bandende 41 ist mit einem als zweiarmiger Hebel, beispielsweise Winkelhebel ausgebildeten Betätigungselement 18 verbunden. Das Betätigungselement 18 ist schwenkbar am Handgriffgehäuse in einer Hebelachse 38 gelagert. Auf den einen Hebelarm des Betätigungselementes 18 wirkt eine Feder 37 im dem Sinne, dass das Reibband 39, welches mit dem anderen Hebelarm verbunden ist, gespannt und damit im Anlagebereich fest auf den Wickelkörper 32 gedrückt wird. Aufgrund der zwischen dem Wickelkörper 32 und dem Reibband 39 wirkenden Haftreibung wird der Wickelkörper 32 und damit der Schieber 14 gegen die Kraft der Vorspannfeder 39 in der gewünschten Position arretiert. Die Feder 37, welche zwischen dem Handgriffgehäuse und dem einen Hebelarm des Betätigungselementes 18 wirkt, vermittelt dabei die erforderliche Bremskraft bzw. Haltekraft.

Beim Betätigen des Betätigungselementes 18 gegen die Kraft der Feder 37 wird der Reibschluss zwischen dem Reibband 39 und dem rollenförmigen Wickelkörper 32 verringert oder gelöst, so dass der Schieber 14 aufgrund der Vorspannkraft der Feder 29 in Richtung zur vorderen Endstellung bewegt oder in seine vordere Endstellung zurückgebracht werden kann. Dabei wird das distale Sondenende 28 mit dem übrigen Sondenteil in axialer Richtung ausgerichtet. Ferner ist es möglich, entgegen der Kraft der Vorspannfeder 29 das Zugelement 33 um einen weiteren Schwenkwinkelbereich auf den Wickelkörper 32 aufzuwickeln, wobei der Schieber 14 zum rückwärtigen (proximalen) Ende des Handgriffs 3 zu bewegt wird. Das distale Sondenende 28 wird dann um einen entsprechenden Betrag weiter verbogen. Durch Loslassen des Betätigungselementes 18 wird aufgrund der von der Feder 37 über das Bremsband 39 vermittelten Haltekraft der Schieber 14 in der neuen gewünschten Position arretiert. Es ist natürlich auch möglich, auf das Betätigungselement 17 eine Betätigungskraft auszuüben, welche die Haftreibung zwischen dem Bremsband 39 und dem Wickelkörper 32 überwindet und in der gewünschten Position die Verstellung des Betätigungselementes 17 zu beenden.

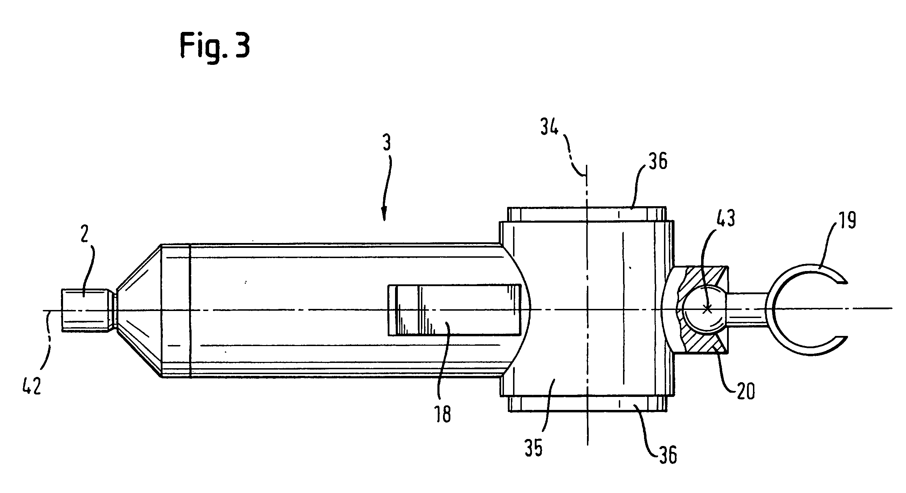

Wie insbesondere aus den Fig. 1 bis 3 zu ersehen ist, sind das Gehäuse des Handgriffs 3, die hohlzylindrische Lagerung 35 des Wickelkörpers 32 am Handgriffgehäuse, die beiden Betätigungselemente 17 und 18 derart ausgebildet, dass sie bezüglich einer Mittelebene 42, welche durch das Gehäuse des Handgriffs 3 gelegt wird, symmetrisch ausgebildet sind. Der Handgriff ist daher sowohl für Linkshänder als auch für Rechtshänder geeignet.

Ferner kann am Handgriff 3, insbesondere am hinteren (proximalen) Ende ein Okularhalter 19 schwenkbar angeordnet sein. Zur schwenkbaren Lagerung ist vorzugsweise ein Gelenk 20, welches insbesondere als Kugelgelenk ausgebildet ist, vorgesehen. Ein Mittelpunkt 43 des Kugelgelenkes liegt vorzugsweise in der Mittelebene 42, so dass sowohl für Rechts- als auch für Linkshänder gleiche Schwenkbedingungen zum Verschenken des Okularhalters 19 in eine geeignete Position vorhanden sind.

Wie die Fig. 4 zeigt, wird die Kathetersonde 1 mittels des lösbaren Verschlusses 2 nur über die Führungseinrichtung 12 für das Steuerelement 13 mit dem Handgriff 3 drehfest verbunden. Die übrigen Ausgänge 9, 10 und 11 sind unabhängig vom Handgriff 3 und können unmittelbar über geeignete Verbindungsmittel mit zugeordneten Endgeräten verbunden werden. Beispielsweise kann der proximale Lumenausgang 9 für den Spül-/Saugkanal mittels eines entsprechenden Anschlussstückes 44 an eine nicht näher dargestellte Spül-/Saugeinrichtung angeschlossen werden. Ferner kann durch den proximalen Lumenausgang 10 in das Arbeitslumen 7 ein chirurgisches Werkzeug, beispielsweise ein Bohrer mit flexibler Welle, wie beispielsweise aus DE 101 078 156 A1 bekannt, oder ein Werkzeug zur Fremdkörperentfernung und Fremdkörperzerkleinerung, insbesondere für den Einsatz bei der mechanischen Lithotripsie entfernbar eingesetzt werden.

Ferner kann über den Lumenausgang 10 in das Arbeitslumen 7 ein Lichtleiterstrang für Laserstrahlung zur Laserstrahlbehandlung am Zielort eingeführt werden. Nach der Behandlung können die jeweiligen chirurgischen Werkzeuge aus dem Arbeitslumen 7 zur Wartung, beispielsweise zur Dekontaminationsbehandlung entfernt werden.

Durch den Lumenausgang 11 kann die Optik 6, welche eine Beleuchtungs- und eine Beobachtungsoptik beinhaltet, in das Optiklumen 4 der Sonde 1 eingesetzt werden. Für die beiden Stränge der Beleuchtungsoptik 22 und der Beobachtungsoptik 23 können auch separate Optiklumina 4 vorgesehen sein. Das jeweilige distale Ende des Optiklumens 4 ist mit einer lichtdurchlässigen Abdeckung 5 hermetisch abgeschlossen. Auf diese Weise wird eine Kontamination der Optik 6 am Zielort verhindert. Der übrige Teil der Optik 6 ist durch die von der Kathetersonde 1 gebildeten Ummantelung gegen Kontamination geschützt. Die Optik 6 kann beispielsweise mit Hilfe eines auf den Optikausgang 11 aufgesetzten Optikschiebers 26 bis zur lichtdurchlässigen Abdeckung 5, welche als Glasscheibe ausgebildet sein kann, im Optikkanal 4 nach vorne verschoben werden. Beim Verbiegen des distalen Sondenendes 28 kann mit Hilfe des Optikschiebers 26 die Optik 6 entsprechend nachgestellt werden und beim Zurückbiegen in die ausgerichtete Position wieder zurückgezogen werden. Der Optikschieber 26 kann beispielsweise wie in DE 199 56 516 A1 beschrieben ausgebildet sein. Im Optikschieber 26 kann auch eine federnde Vorspannkraft wirken, welche ein selbsttätiges Vorschieben der Optik 6 beim Verbiegen des distalen Sondenendes 28 gewährleistet. Beim Zurückbringen des Sondenendes in seine gerade Position wird die Optik 6 gegen die Vorspannkraft zurückgeschoben. Hierdurch ist gewährleistet, dass die Optik 6 mit ihrem distalen Ende immer an der Abdeckung bzw. Abdichtung 5 gegebenenfalls mit einer bestimmten Andrückkraft anliegt.

Das proximale Ende des Stranges der Beleuchtungsoptik 22 kann mit einer Beleuchtungseinrichtung 24 verbunden sein. Das proximale Ende des Stranges der Beobachtungsoptik 23 kann mit einer Beobachtungseinrichtung 25 verbunden sein. Die Beobachtungseinrichtung 25 kann beispielsweise ein am Okularhalter 19 befestigbares Okular sein. Ferner kann die Beobachtungseinrichtung 25 als Kamera-/Monitorsystem ausgebildet sein. Wie die Fig. 4 zeigt, sind die Ausgänge 9, 10 und 11 nicht mit dem Handgriff 3 verbunden.

Da die Kathetersonde 1 und das proximale Sondenansatzstück 8 mit den zugehörigen Lumenausgängen 9, 10 und 11 sowie die Führungseinrichtung 12 als Einmalteil, beispielsweise in Spritzgrußtechnik hergestellt werden können, verbleibt für die Dekontaminationsbehandlung nur noch das jeweils zum Einsatz gebrachte chirurgische Werkzeug. Der Handgriff 3 ist so ausgebildet, dass er mit verschiedenen Kathetersonden, die ein Steuerelement nach Art des Steuerelementes 13 aufweisen, verwendet werden.

Beispielsweise kann das beschriebene Endoskop zur Herzkranzgefäßendoskopie verwendet werden, wobei wie beispielsweise aus US-A-4,762,120 bekannt, die Kathetersonde in ihrem distalen Sondenbereich mit einem Ballon ausgestattet sein kann.

Ferner kann der Handgriff mit Kathetersonden für den Einsatz bei der Cholangioskopie und mechanischen Lithotripsie, insbesondere der perkutanen mechanischen Lithotripsie zum Einsatz gebracht werden.

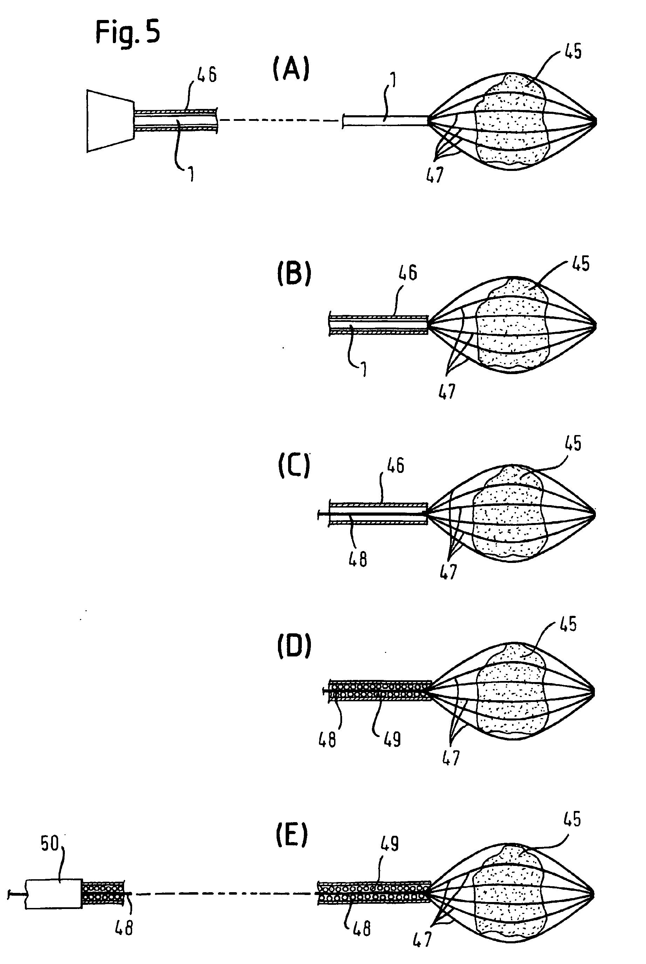

Zur Durchführung mechanischer Lithotripsie wird, wie anhand der Figur 5 erläutert wird, die Kathetersonde 1 mit Beobachtung über die Optik 6 und die Beobachtungseinrichtung 23 bis zum Ort des zu entfernenden Fremdkörpers 45, insbesondere Steins, geführt. Über den nicht in den Patientenkörper eingeführten Sondenteil in der Nähe des proximalen Endes ist ein Überrohr 46 geschoben. Dieses Überrohr 46 hat eine Länge, welche zumindest der in den Körper eingeschobenen Sondenlängen entspricht. Mit Hilfe eines Greifwerkzeugs 47, beispielsweise Körbchens, welches durch das Arbeitslumen 7 geschoben ist und über das distale Ende des Arbeitslumens ragt, wird der Fremdkörper bzw. Stein erfasst Schritt (A). Anschließend wird das Überrohr 46, welches vorzugsweise ebenfalls flexibel und biegbar ausgebildet ist, bis zum Anschlag an den erfassten Stein 45 geschoben, wobei ein proximales Endstück des Überrohres 46 über die Körperöffnung, durch welche die Kathetersonde 1 eingeführt wurde, übersteht Schritt (B). Anschließend wird die Kathetersonde 1 aus dem Körper des Patienten entfernt, wobei das Überrohr 45 in seiner am Körbchen 47 mit dem erfassten Stein 45 anschlagenden Stellung belassen wird Schritt (C). Ein mit dem Körbchen verbundener Zugdraht 48 oder verbundenes Zugmittel ragt durch das Überrohr 46 und ragt aus dem proximalen Ende des Überrohres 46. Nach dem Entfernen der Kathetersonde 1 wird bis zum Anschlag an das Körbchen 47 durch den Hohlraum des Überrohres 46 eine Spirale 49 oder gleichwirkendes hohles Abstützelement, welche bzw. welches in bekannter Weise bei der mechanischen Lithotripsie verwendet wird, über den Zugdraht 48, welcher mit dem Körbchen verbunden ist, bis zum Anschlag an das Körbchen geschoben Schritt (D). Mit Hilfe einer bekannten Zugeinrichtung 50, welche am proximalen Ende des Zugdrahtes angreift und am proximalen Ende der Spirale abgestützt wird, wird auf das Körbchen 47 über den Zugdraht 48 ein Zug ausgeübt, der zur Zertrümmerung des erfassten Fremdkörpers 45, insbesondere Steins führt Schritt (E). Dabei werden Spirale 49 und Zugdraht 48 relativ zueinander bewegt, wobei der zu zerkleinernde Fremdkörper 45 gegen das distale Ende der Spirale 49 gedrückt wird. Auf diese Weise lässt sich in vorteilhafter Weise eine perkutane mechanische Lithotripsie durchführen. Auch in Urethrorenoskopie und Cholangioskopie kann die beschriebene mechanische Lithotripsie durchgeführt werden. Für die Einrichtung zur Durchführung der mechanischen Lithotripsie können bekannte Einrichtungen, wie sie beispielsweise aus DE 102 41 946 A1 oder DE 199 55 614 C1 bekannt sind, verwendet werden.

### [Bezugszeichenliste]

- 1: Kathetersonde
- 2: lösbarer Verschluss
- 3: Handgriff
- 4: Optiklumen
- 5: lichtdurchlässige Abdeckung (Abdichtung)
- 6: Optik
- 7: Arbeitslumen
- 8: proximales Sondenansatzstück
- 9: proximaler Lumenausgang
- 10: proximaler Lumenausgang
- 11: proximaler Lumenausgang
- 12: Führungseinrichtung für Steuerelement
- 13: Steuerelement
- 14: Schieber
- 15: Kurbeltrieb
- 16: Arretiereinrichtung
- 17: Betätigungselement für Schieberbewegung
- 18: Betätigungselement für Schieberarretierung
- 19: Okularhalter
- 20: Gelenk (Kugelgelenk)
- 21: lösbares Befestigungsmittel (z.B. Klemm- schraube)
- 22: Beleuchtungsoptik
- 23: Beobachtungsoptik
- 24: Beleuchtungseinrichtung
- 25: Beobachtungseinrichtung
- 26: Optikschieber
- 27: Spüllumen (Spülkanal)
- 28: distales Sondenende
- 29: Vorspannkraft (Feder)
- 30: Längsschlitz
- 31: Kurbeltrieb
- 32: Wickelkörper
- 33: Zugmittel
- 34: Achse
- 35: hohlzylindrisches Lager
- 36: Endscheiben
- 37: Feder
- 38: Hebelachse
- 39: Reibband
- 40: Bandende
- 41: Bandende
- 42: Mittelebene
- 43: Mittelpunkt des Kugelgelenks
- 44: Anschlussstück
- 45: Fremdkörper (Stein)
- 46: Überrohr
- 47: Körbchen
- 48: Zugdraht
- 49: Spirale
- 50: Zugeinrichtung

## Patentansprüche

1. Endoskop mit einer flexiblen und mehrere Lumen (4, 7, 22) aufweisenden Kathetersonde (1), an deren proximalem Ende zugeordnete Lumenausgänge (9, 10, 11) vorgesehen sind, einem Handgriff (3), welcher am proximalen Sondenende vorgesehen ist, einer Optik (6), welche in wenigstens einem Optiklumen (4) der Kathetersonde (1) vorgesehen ist, wobei das distale Ende des Optiklumens (4) eine lichtdurchlässige Abdichtung (5) aufweist und die Optik (6) im Optiklumen (4) verschiebbar und aus dem Optiklume(4) entfernbar angeordnet ist, wenigstens einem Arbeitslumen (7) für ein chirurgisches Werkzeug und einer Steuereinrichtung, welche am distalen Sondenende oder in der Nähe davon zum Biegen des Sondenendes befestigt ist und in axialer Richtung an der Sonde beweglich geführt ist, wobei die Steuereinrichtung mittels eines lösbaren Befestigungsmittels (21) mit einem im Handgriff (3) geführten Schieber (14) zu verbinden ist, **dadurch gekennzeichnet, dass** am proximalen Ende der Kathetersonde (1) ein drehsteifes Sondenansatzstück (8) vorgesehen ist, welches die mehreren Lumenausgänge (9, 10, 11) für die Sondenlumen (4, 7, 21) und eine drehsteife röhrchenförmige Führungseinrichtung (12), in dessen Röhrchenhohlraum ein längliches Steuerelement (13) zum Biegen des Sondenendes geführt ist, aufweist und dass die drehsteife Führungseinrichtung (12) mittels eines lösbaren Verschlusses (2) drehfest mit dem Handgriff (3) für eine drehfeste Verbindung zwischen Kathetersonde (1) und Handgriff (3) zu verbinden ist.

2. Endoskop nach Anspruch 1,
**dadurch gekennzeichnet, dass** das chirurgische Werkzeug aus dem wenigstens einen Arbeitslumen (7) entfernbar ist.

3. Endoskop nach Anspruch 1 oder 2,
**dadurch gekennzeichnet, dass** die Kathetersonde (1) als Einmalteil ausgebildet ist.

4. Endoskop nach einem der Ansprüche 1 bis 3,
**dadurch gekennzeichnet, dass** die Kathetersonde (1) als Spritzgussteil oder Extruderteil ausgebildet ist.

5. Endoskop nach einem der Ansprüche 1 bis 4,
**dadurch gekennzeichnet, dass** der Schieber (14) mittels eines am Handgriffgehäuse geführten Betätigungselementes gegen eine Vorspannkraft (29) zu verschieben ist.

6. Endoskop nach einem der Ansprüche 1 bis 5,
**dadurch gekennzeichnet, dass** der Schieber (14) mittels einer eine Drehbewegung in eine axiale Linearbewegung umsetzenden Einrichtung, insbesondere mittels eines Kurbeltriebs (15) verschiebbar am Handgriffgehäuse gelagert ist.

7. Endoskop nach einem der Ansprüche 1 bis 6,
**dadurch gekennzeichnet, dass** der Schieber (14) mittels einer Arretiereinrichtung (16) in verschiedenen Positionen am Handgriffgehäuse arretierbar ist.

8. Endoskop nach einem der Ansprüche 1 bis 7,
**dadurch gekennzeichnet, dass** das Gehäuse des Handgriffs (3) und am Handgriff angeordnete Betätigungselemente (17, 18) zur Betätigung der Schieberbewegung und zur Arretierung der Schieberbewegung bezüglich einer durch den Handgriff (3) verlaufenden Mittelebene (42) symmetrisch ausgebildet sind.

9. Endoskop nach einem der Ansprüche 1 bis 8,
**dadurch gekennzeichnet, dass** am proximalen Ende des Handgriffgehäuses ein Okularhalter (19) in einem Gelenk (20), welches insbesondere als Kugelgelenk ausgebildet ist, angeordnet ist.

10. Endoskop nach den Ansprüchen 8 und 9,
**dadurch gekennzeichnet, dass** der Mittelpunkt (43) des Kugelgelenks in der Mittelebene (42) liegt.

11. Endoskop nach einem der Ansprüche 1 bis 10,
**dadurch gekennzeichnet, dass** die Drehachse (34) des Kurbeltriebs (15) senkrecht zur Mittelebene (42) verläuft.

12. Endoskop nach einem der Ansprüche 1 bis 11,
**dadurch gekennzeichnet, dass** der Kurbeltrieb (15) in einem hohlzylindrischen Lager (35), welches Bestandteil des Handgriffgehäuses ist, um die Drehachse (24) drehbar gelagert ist.

13. Endoskop nach einem der Ansprüche 1 bis 12,
**dadurch gekennzeichnet, dass** die Lumenausgänge (9, 10, 11) für die mehreren Sondenlumen (4, 7, 27) unabhängig vom Handgriff (3) und außerhalb des Handgriffs (3) an zugeordnete Endgeräte anschließbar sind.

14. Endoskop nach einem der vorherigen Ansprüche, welches mit einer Einrichtung zur mechanischen Lithotripsie und einem über die Kathetersonde (1) schiebbaren Überrohr (46) kombiniert ist.

15. Endoskop nach Anspruch 14, bei dem das Überrohr (46) länger ausgebildet ist als die in den Patientenkörper einzuschiebende Kathetersondenlänge.

## Claims

1. Endoscope comprising: a flexible catheter probe (1) having a plurality of lumina (4, 7, 22), wherein dedicated lumen outlets (9, 10,11) are provided at a proximal end of the probe (1); a hand grip (3) which is provided at the proximal end of the probe; an optic device (6) which is provided at least in one optic lumen (4) of the catheter probe (1), the distal end of the optic lumen (4) having a transparent seal (5) and the optic device (6) being slidable within the optic lumen (4) and being removable from the optic lumen (4); at least one working lumen (7) for a surgical tool; and a controller being attached to the distal end of the probe or in the vicinity thereof for bending of the end of the probe, and being guided movable in an axial direction, wherein the controller is to be connected to a slide (14) guided within the hand grip (3) by means of a detachable fixing means (21),
**characterized in that** a torsion proof probe extension (8) is provided at the proximal end of the catheter probe (1), which comprises the plurality of lumen outlets (9, 10, 11) for the probe lumina (4, 7, 21) and a torsion proof tube-shaped guide device (12), in whose tube cavity an elongated control element (13) for bending the end of the probe is guided, and that the torsion proof guide device (12) is to be connected in a torque proof manner to the hand grip (3) by means of a detachable lock (2) for a torque proof connection between the catheter probe (1) and the hand grip (3).

2. Endoscope according to claim 1,
**characterized in that** the surgical tool is removable from the at least one working lumen (7).

3. Endoscope according to claim 1 or 2,
**characterized in that** the catheter probe (1) is formed as a disposable element.

4. Endoscope according to any one of the claims 1 to 3,
**characterized in that** the catheter probe (1) is formed as a die-casting element or an extruded element.

5. Endoscope according to any one of the claims 1 to 4,
**characterized in that** the slide (14) is to be moved against a preload force (29) by means of a actuating element guided at the hand grip housing.

6. Endoscope according to any one of the claims 1 to 5,
**characterized in that** the slide (14) being supported at the hand grip housing by means of a device converting a rotary motion into a axial linear motion, in particular by means of a crank drive (15).

7. Endoscope according to any one of the claims 1 to 6,
**characterized in that** the slide (14) is lockable in several positions at the hand grip housing by means of a locking device (16).

8. Endoscope according to any one of the claims 1 to 7,
**characterized in that** the housing of the hand grip (3) and actuating elements (17, 18) arranged at the hand grip for actuating the slide motion and locking the slide motion are formed in a symmetrical manner with respect to a center plane (42) extending through the hand grip (3).

9. Endoscope according to any one of the claims 1 to 8,
**characterized in that** an eyepiece holder (19) is arranged at the proximal end of the hand grip in a joint (20) which in particular is formed as a ball joint.

10. Endoscope according to claim 8 or 9,
**characterized in that** the center (43) of the ball joint being located within the center plane (42).

11. Endoscope according to any one of the claims 1 to 10,
**characterized in that** the rotary axis (34) of the crank drive (15) extends perpendicular to the center plane (42).

12. Endoscope according to any one of the claims 1 to 11,
**characterized in that** the crank drive (15) being supported rotatable around the rotary axis (24) in a hollow cylindrical bearing (35) which is part of the hand grip housing.

13. Endoscope according to any one of the claims 1 to 12,
**characterized in that** the lumen outlets (9, 10, 11) for the plurality of probe lumina (4, 7, 27) being connectable to dedicated terminal equipments, independent from the hand grip (3) and outside of the hand grip (3).

14. Endoscope according to any one of the preceding claims, being combined with a means for mechanical lithotripsy and a cover tube (46) slidable over the catheter probe (1).

15. Endoscope according to claim 14, wherein the cover tube (46) formed to be longer than the catheter probe length to be inserted into the body of a patient.

## Revendications

1. Endoscope comprenant un cathéter (1) flexible, qui présente plusieurs lumens (4, 7, 27) et sur l'extrémité proximale duquel sont prévues des sorties de lumens (9, 10, 11) associées, une poignée (3) prévue sur l'extrémité proximale de la sonde, une optique (6) prévue dans au moins un lumen optique (4) du cathéter (1), l'extrémité distale du lumen optique (4) présentant une étanchéité transparente (5) et l'optique (6) étant disposée avec une possibilité de déplacement dans le lumen optique (4) et de retrait du lumen (4), au moins un lumen de travail (7) pour un outil chirurgical et un dispositif de commande, qui est fixé sur l'extrémité distale de la sonde ou à proximité de cette dernière pour la flexion de l'extrémité de sonde et est guidé mobile dans la direction axiale sur la sonde, le dispositif de commande pouvant être assemblé à l'aide d'un moyen de fixation amovible (21) avec une coulisse (14) guidée dans la poignée (3), **caractérisé en ce qu'**il est prévu, sur l'extrémité proximale du cathéter (1), un embout de sonde (8) résistant à la torsion, qui présente les multiples sorties de lumens (9, 10, 11) pour les lumens (4, 7, 27) de la sonde et un dispositif de guidage (12) tubulaire résistant à la torsion, dans l'espace creux tubulaire duquel est guidé un élément de commande (13) allongé pour la flexion de l'extrémité de sonde, et que le dispositif de guidage (12) résistant à la torsion peut être assemblé de façon solidaire, au moyen d'une fermeture amovible (2), avec la poignée (3) pour un assemblage résistant à la torsion entre le cathéter (1) et la poignée (3).

2. Endoscope suivant la revendication 1, **caractérisé en ce que** l'outil chirurgical peut être retiré du au moins un lumen de travail (7).

3. Endoscope suivant l'une des revendications 1 et 2, **caractérisé en ce que** le cathéter (1) est réalisé sous forme de pièce à usage unique.

4. Endoscope suivant l'une des revendications 1 à 3, **caractérisé en ce que** le cathéter (1) est réalisé sous forme de pièce moulée par injection ou de pièce extrudée.

5. Endoscope suivant l'une des revendications 1 à 4, **caractérisé en ce que** la coulisse (14) peut être déplacée contre une force de précontrainte (29) au moyen d'un élément d'actionnement guidé sur le boîtier de poignée.

6. Endoscope suivant l'une des revendications 1 à 5, **caractérisé en ce que** la coulisse (14) est montée avec une possibilité de déplacement sur le boîtier de poignée au moyen d'un dispositif de conversion d'un mouvement de rotation en un mouvement linéaire axial, en particulier au moyen d'un mécanisme à manivelle (15).

7. Endoscope suivant l'une des revendications 1 à 6, **caractérisé en ce que** la coulisse (14) peut être bloquée dans différentes positions sur le boîtier de poignée au moyen d'un dispositif d'arrêt (16).

8. Endoscope suivant l'une des revendications 1 à 7, **caractérisé en ce que** le boîtier de la poignée (3) et des éléments d'actionnement (17, 18) disposés sur la poignée, destinés à l'actionnement du déplacement de la coulisse et à l'arrêt de ce dernier, ont une réalisation symétrique par rapport à un plan médian (42), qui traverse la poignée (3).

9. Endoscope suivant l'une des revendications 1 à 8, **caractérisé en ce qu'**un support d'oculaire (19) est disposé sur l'extrémité proximale du boîtier de poignée dans une articulation (20), réalisée en particulier sous forme d'articulation sphérique.

10. Endoscope suivant les revendications 8 et 9, **caractérisé en ce que** le centre (43) de l'articulation sphérique se situe dans le plan médian (42).

11. Endoscope suivant l'une des revendications 1 à 10, **caractérisé en ce que** l'axe de rotation (34) du mécanisme à manivelle (15) s'étend perpendiculairement au plan médian (42).

12. Endoscope suivant l'une des revendications 1 à 11, **caractérisé en ce que** le mécanisme à manivelle (15) est monté tournant autour de l'axe de rotation (34) dans un palier cylindrique et creux (35), qui fait partie du boîtier de poignée.

13. Endoscope suivant l'une des revendications 1 à 12, **caractérisé en ce que** les sorties de lumens (9, 10, 11) pour les multiples lumens (4, 7, 27) de la sonde peuvent être raccordées à des terminaux associés indépendamment de la poignée (3) et à l'extérieur de la poignée (3).

14. Endoscope suivant l'une des revendications précédentes, qui est combiné à un dispositif de lithotripsie mécanique et à un sur-tube (46) mobile sur le cathéter (11).

15. Endoscope suivant la revendication 14, dans lequel le sur-tube (46) a une réalisation plus longue que la longueur du cathéter à insérer dans le corps du patient.
